# EUROPEAN PATENT SPECIFICATION

(11) **EP 2 419 485 B1**
(45) Date of publication and mention of the grant of the patent: **23.05.2018**
(21) Application number: 10765187.9
(22) Date of filing: 15.04.2010
(51) Int. Cl.: C09J 183/04, C09J 133/08, C09J 7/00, B32B 5/18, B32B 25/04, B32B 25/10, B32B 25/20, B32B 27/30, A61F 13/02, A61L 15/58, C09J 133/04

(54) **SILICONE GEL ADHESIVE CONSTRUCTION**
HAFTMEDIUM MIT SILIKONGEL
CONSTRUCTION ADHÉSIVE EN GEL DE SILICONE

(30) Priority: 17.04.2009 US 170447 P
(43) Date of publication of application: 22.02.2012
(73) Proprietor: 3M Innovative Properties Company, St. Paul, MN 55133-3427 (US)
(72) Inventor: KANTNER, Steven, S., Saint Paul, Minnesota 55133-3427 (US); LEWIS, Terry, W., Woodbury, Minnesota 55125 (US); BROWN, Mary, L., Saint Paul, Minnesota 55133-3427 (US)
(74) Representative: Herzog, Fiesser & Partner Patentanwälte PartG mbB
(86) International application number: PCT/US2010/031250
(87) International publication number: WO 2010/121033

(56) References cited:
- WO-A1-2004/082935
- WO-A1-2005/051442
- WO-A1-2005/102403
- WO-A1-2006/081403
- WO-A1-2008/057155
- WO-A2-2007/113597
- US-A- 4 177 301
- US-A- 4 838 253
- US-A- 5 496 603
- US-A- 6 103 369
- US-A1- 2005 169 977
- US-A1- 2005 202 073
- US-A1- 2007 128 263
- US-B1- 6 441 092
- US-B1- 6 794 030
- "Silicones", 15 April 2003 (2003-04-15), ENCYCLOPEDIA OF POLYMER SCIENCE AND TECHNO, WILEY, US, PAGE(S) 765 - 841, XP007918236, * pages 779-782 *

## Description

### FIELD

This invention relates to silicone gel adhesive constructions that are useful, for example as medical tapes or dressings.

### BACKGROUND

It is desirable to use silicone gel adhesives in medical tape and dressing constructions because they provide good adhesion to skin with gentle removal force and the ability to re-stick. In addition, being crosslinked gels, they do not flow around hair and therefore do not pull hair on removal.

Uncured silicone gels have low viscosity (for example, between about 1000 and 6000 mPa·s), however, which makes coating one side of a porous substrate very difficult. The silicone, which has a low surface energy, readily wets most surfaces and migrates into and through porous substrates. In medical tape and dressing constructions, such migration is undesirable because it leads to staining and/or tackiness on the backside of the construction. It also results in wasted material lost within the substrate.

One approach to overcoming this problem, as disclosed, for example, in U.S. Patent No. 7,161,056, is to extrude silicone gel precursor onto a planar molding surface, which is then heated. An absorbent material can be laminated to the top surface after the silicone has started to crosslink, but before it fully crosslinks. This method is claimed to be effective at forming a good bond between the absorbent material and the cured silicone gel without flow of the silicone compound into the open cavities of the absorbent material. But, this method requires careful control of time and temperature to ensure that the silicone reaches a uniform level of cure, including cross-web, prior to contacting the absorbent material. It can therefore be very difficult to avoid randomly distributed areas of variable cure.

If silicone gel precursor is too fully cured prior to lamination, inadequate adhesion between the two materials results. By its nature, a silicone gel adhesive does not have high adhesion to surfaces. Hence, adhesion of cured silicone gel adhesive to a surface will be low, particularly if the surface is a woven, non-woven, or knitted fabric-like material wherein total contact between the silicone gel and the fabric is low.

Approaches to improve this so-called "two-bond" adhesion include modification of the silicone gel by incorporation of hydroxyl-substituted siloxane resin (see, for example, U.S. Patent Application Pub. No. 2007/0202245) or by coating the substrate or cured silicone gel with primer selected from titanate materials, zirconate materials, Si-H containing siloxanes and platinum materials (see, for example, WO 2007/113597 or U.S. Patent Application Pub. No. 2007/0042108).

Nevertheless, silicone gel adhesive products on the market tend to have relatively thick constructions. For example, they tend to include silicone gels at thicknesses of 6 mils or more. They may also include netting within the gel or a scrim attached to the adhesive side of the film backing to provide mechanical anchorage, necessitating thicker constructions. Such thick constructions can leave more of a tacky edge exposed, which can catch or clothing and the like causing the tape or dressing to lift. In addition, although silicone adhesives have high moisture vapor transmission rates (MVTR), MVTR is inversely proportional to thickness. Thus, thicker constructions can have relatively poor actual MVTR. Placing a tape or dressing with an MVTR lower than the MVTR of skin (approximately 200 to 300 g/m²/24 hours) on skin allows moisture to accumulate at the skin/adhesive interface, which reduces bond strength. Such moisture can also macerate the skin, weakening it and leading to greater damage when the tape is removed. It is therefore desirable for a medical tape or dressing to have an MVTR of at least about 300 g/m²/24 hours.

### SUMMARY

In view of the foregoing, we recognize that there is a need in the art for improved medical tape or dressing constructions with skin-contacting silicone gel adhesives on porous backings as claimed such as woven, non-woven, and knitted fabric-like backings, and for methods of making such constructions. Specifically, we recognize that the constructions should include silicone gel adhesives that are well adhered to the porous backing.

Briefly, in one aspect, the present invention provides a silicone gel adhesive construction comprising (a) a porous backing, (b) an acrylic copolymer pressure sensitive adhesive layer on at least a portion of one side of the porous backing, and (c) a cured silicone gel adhesive on the pressure sensitive adhesive layer.

In another aspect, the present invention provides methods for making silicone gel adhesive constructions. A first method comprises substantially sealing at least a portion of one side of a porous backing with an acrylic copolymer pressure sensitive adhesive, coating a silicone adhesive gel precursor on the pressure sensitive adhesive, and curing the silicone adhesive gel precursor. A second method comprises substantially sealing at least a portion of one side of a porous backing with an acrylic copolymer pressure sensitive adhesive, and laminating a cured silicone gel adhesive to the pressure sensitive adhesive.

The silicone gel adhesive constructions of the present invention are useful as medical tapes and dressings. The constructions include silicone gel adhesives that are well adhered to the porous backing. In addition, in at least some embodiments of the silicone gel adhesive constructions of the invention, the constructions have a low profile with minimal tacky edge exposed. Many of the constructions of the invention also have MVTRs of at least about 300 g/m²/24 hours (preferably, at least about 600 g/m²/24 hours; more preferably, at least about 1000 g/m²/24 hours).

The silicone gel adhesive constructions of the invention and the methods of the invention therefore meet the need in the art for improved medical tape or dressing constructions with skin-contacting silicone gel adhesives on porous backings as claimed such as woven, non-woven, and knitted fabric-like backings, and for methods of making such constructions.

### DETAILED DESCRIPTION

### Porous Substrate

The silicone gel adhesive constructions of the invention comprise a porous backing as claimed. Preferably, the porous backing comprises a material that is soft, elastic, and conformable.

For example, the porous backing can be made of fabrics, nonwoven fabrics, melt-blown webs, spun-bonded webs, thermal-bonded webs, spun-laced webs, paper, and thermally-embossed nonwoven fabrics, and those described in U.S. Pat. No. 5,496,603. More precisely, examples of the substrate may be woven fabrics, knitted fabrics or non-woven fabrics of an organic polymer such as cotton, polyvinyl alcohol or cellulose; paper; and perforated films of polyvinyl alcohol. If desired, the substrate may be processed for water repellency with a known water repellent. The substrate may be elastic or non-elastic. Preferably, the substrate has good air permeability and moisture permeability and has good elasticity.

Preferred porous backings include elastic cotton fabrics (woven fabrics) or nonwoven fabrics, and porous backings comprising a non-woven melt-blown polyurethane material. In one preferred embodiment, the porous backing comprises a melt blown polyurethane web as described in Example 1 of U.S. Patent No. 7,066,182 blown onto a bleached, densified 54.5# paper.

Examples of other useful woven and non-woven backings are disclosed in U.S. Patent No. 6,497,949.

### Acrylic Copolymer Pressure Sensitive Adhesive

The porous backing is at least 75% sealed on one side with a thin coating of an acrylic copolymer pressure sensitive adhesive (PSA). Acrylic copolymer PSAs useful in this invention include those with relatively good MVTR, above about 300 g/m²/24 hr (preferably, above 600 g/m²/24 hr). Such PSAs are known in the art and include a majority of a rubbery monomer such as, for example, 2-ethylhexyl acrylate or butyl acrylate, and a minor amount of a polar monomer such as, for example, acrylic acid or acrylamide. Blends of these PSAs with polar additives are also useful.

The acrylic copolymer PSA can be applied by coating a water-based emulsion acrylic copolymer, a 100% solids hot-melt acrylic copolymer, or a solvent-based solution acrylic copolymer. An example of a suitable hot-melt acrylic copolymer is disclosed, for example, in U.S. Patent No. 6,441,092. It comprises a blend of 2-ethylhexyl acrylate/acrylic acid/4-acryloyl-oxybenzophenone (ABP) and Avalure™ AC 210 acrylate copolymer (for example, in an 85/15 weight ratio). An example of a suitable water-based acrylic copolymer is described in U.S. Patent No. 4,973,513 as adhesive BBB.

Emulsion polymers and hot-melt polymers are generally preferred due to environmental considerations and the sensitivity of some backing materials, including polyurethanes, to solvents. The acrylic copolymer can be coated directly on the porous backing or it can be coated onto a release liner and then laminated to the backing (optionally after drying). Coating directly onto the porous backing is generally preferred in order to minimize the number of steps and to eliminate the need for a process liner. However, in some situations, for example, when solvent-based acrylic copolymer PSAs are used with solvent-sensitive substrates, the lamination method is preferred.

The coating weight of the acrylic copolymer PSA should be sufficient to prevent excessive pinholes in the coating, which leads to strike-through when silicone gel adhesive precursor is added. The coating weight of the acrylic copolymer PSA on the porous backing is typically from about 15 g/m² to about 80 g/m² (preferably from about 20 g/m² to about 45 g/m²).

### Silicone Gel Adhesive

The silicone gel adhesive constructions of the invention comprise a skin-contacting silicone adhesive gel. Silicone gel adhesive precursor can be coated onto the porous substrate substantially sealed with acrylic copolymer PSA. The silicone gel adhesive precursor can then be cured. Alternatively, the silicone gel adhesive precursor can be cured on a suitable release liner and laminated to the acrylic copolymer PSA coated side of the porous backing.

Silicone gel adhesives are known in the art. As detailed in WO 2008/057155, they are lightly crosslinked silicone polymers that have a viscoelastic, jelly-like consistency. They are formed using a hydrosilation reaction between an alpha-omega vinyl terminated polydimethyl siloxane and a Si-H containing siloxane catalyzed by a platinum catalyst. Further details on their formulation and properties are disclosed, for example, in U.S. Patent Nos. 4,991,574 and 5,145,933.

Suitable silicone gel adhesive precursors are commercially available. Several manufacturers sell versions of these materials based on platinum catalyzed two component addition cure chemistry. Such materials (uncured) typically have viscosities of about 1000 mPa·s to about 6000 mPa·s. Examples of suitable commercially available silicone gel precursors include Blue Star Silicones Silbione™ RT Gel 4317, Dow Corning MG 7-9850 Soft Skin Adhesive (SSA), and Wacker SilGel™ 612, all of which are two component 100% solids platinum catalyzed addition-cure materials.

The coating weight of the silicone gel adhesive typically ranges from about 20 g/m² to about 150 g/m² (preferably, from about 40 g/m² to about 120 g/m²). The silicone gel adhesive coating is typically from about 0.8 to about 6 mils thick. Lower coating weights may not provide adequate adhesion properties to skin while higher coating weights are more expensive, give a higher profile tacky edge, and reduce MVTR.

Surprisingly, the silicone gel forms a high interfacial bond to the acrylic copolymer PSA. Silicones have traditionally been used as release surfaces for a wide variety of acrylic PSAs and contamination of such PSAs with even a small amount of free silicone fluid from an undercured liner is known to greatly reduce tack. As noted in Chapter 24, p. 602, of Donatas Satas, Handbook of Pressure Sensitive Adhesive Technology, 2nd Ed., silicone coatings exhibit low attractive forces for other molecules and are considered to be incompatible with organic polymers. The silicone gel adhesive constructions of the invention, however, possess good two-bond adhesion.

### EXAMPLES

Objects and advantages of this invention are further illustrated by the following examples, but the particular materials and amounts thereof recited in these examples, as well as other conditions and details, should not be construed to unduly limit this invention.

### Materials

### Porous backing

The porous backing used in these examples is a melt blown polyurethane (MBPU) web described in Example 1 of U.S. Patent No. 7,066,182. It was blown onto a bleached, densified 54.5# paper to which it adhered.

### Acrylic Copolymer Barrier Coat

"Hot melt" - a blend of 85 weight percent of 2-ethylhexyl acrylate/acrylic acid/ABP (96.5/3.5/0.05 weight ratio) and 15 weight percent Avalure™ AC 210 Acrylate copolymer as disclosed in U.S. Pat. No. 6,441,092.

"Water-based" - water-based acrylic adhesive described in U.S. Pat. 4,973, 513, Adhesive BBB.

### Silicone Gel Adhesives

Three silicone gel adhesives were used: Blue Star Silicones Silbione™ RT Gel 4317 ("Blue Star"), Dow Corning MG 7-9850 Soft Skin Adhesive ("Dow"), and Wacker SilGel™ 612 ("Wacker"). All are two component 100% solids platinum catalyzed addition-cure materials.

### Preparation of Substrates

The MBPU web was coated with either the hot melt adhesive or the water-based adhesive as follows:
The MBPU was hot melt coated with the adhesive blend at coating weights of 10 grains/4"x 6" (42 g/m²) and 15 grains/4"x 6" (63 g/m²).
The MBPU web was coated with the water-based adhesive using #5, #12, and #22 Mayer rods and dried at 65° C for 10 minutes. The resulting coating weights were relatively independent of the Mayer rod, falling in the range of 5.6 to 6.3 grains/4x6 (23 to 26 g/m²).

When removed from the paper carrier, the uncoated side of the MBPU web had the same appearance and feel as the starting uncoated web.

### Coating with Silicone Gel Adhesives

The two components of the silicone gel adhesives were mixed in equal portions and coated onto the MBPU web substrates using a knife coater with the gap set to either 0.36 or 0.38 mm (14 mil or 15 mil) (nominally 0.03 or 0.05 mm (1 or 2 mil) thicker than the MBPU web on paper carrier). The resulting coatings were placed in a 65° C oven for 7 minutes to cure.

### Tests

Strike-through of the silicone gels was assessed by estimating the % of staining seen on the paper carrier. MVTR was evaluated in a manner analogous to that described in ASTM E 96-80 at 40° C. and expressed in grams transmitted per square meter per day (g/m²/24 hr).

| **Example** | **Barrier Coat** | **Coating Weight** | **Adhesive** | **% Strike Through** | **MVTR** |
|---|---|---|---|---|---|
| Comp 1 | None | 0 | Blue Star | 100% | ND |
| Comp 2 | None | 0 | Dow | 100% | ND |
| Comp 3 | None | 0 | Wacker | 100% | ND |
| 1 | Water-based | # 5 MR | Blue Star | 15-20% | 1500 |
| | | 24 g/m² | | | |
| 2 | Water-based | # 12 MR | Blue Star | 5-10% | 1792 |
| | | 23 g/m² | | | |
| 3 | Water-based | # 22 MR | Blue Star | 3-5% | 1167 |
| | | 25 g/m² | | | |
| 4 | Hot melt | 42 g/m² | Wacker | 3-5% | 652 |
| 5 | Hot melt | 63 g/m² | Wacker | 1% | 326 |

Various modifications and alterations to this invention will become apparent to those skilled in the art without departing from the scope of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A silicone gel adhesive construction comprising:
(a)a porous backing being a woven fabric, a knitted fabric, a nonwoven fabric, a melt-blown web, a spun-bonded web, a thermal-bonded web, a spun-laced web, paper, and a thermally-embossed nonwoven fabric,
(b)an acrylic copolymer pressure sensitive adhesive layer on and sealing at least 75% of one side of the porous backing, and
(c)a cured silicone gel adhesive on the pressure sensitive adhesive layer, wherein the silicone gel adhesive is formed from a hydrosilation reaction between an alpha-omega vinyl terminated polydimethyl siloxane and a Si-H containing siloxane catalyzed by a platinum catalyst.

2. The silicone gel adhesive construction of claim 1 wherein the construction has a moisture vapor transmission rate according to ASTM E 96-80 at 40° C of at least 300 g/m²/24 hours.

3. The silicone gel adhesive construction of claim 1 wherein the coating weight of the acrylic copolymer pressure sensitive adhesive layer is from 15 g/m² to 80 g/m².

4. The silicone gel adhesive construction of claim 1 wherein the coating weight of the silicone gel adhesive is from 20 g/m² to 150 g/m².

5. The silicone gel adhesive construction of claim 1 wherein the silicone gel adhesive is from 0.02 millimeters (0.8 mils) to 0.15 millimeters (6 mils) thick.

6. The silicone gel adhesive construction of claim 1 wherein the pressure sensitive adhesive comprises a majority of 2-ethylhexyl acrylate or butyl acrylate and a minor amount of acrylic acid or acrylamide.

7. A method for making a silicone gel adhesive construction comprising:
(a) sealing at least 75% of one side of a porous backing with an acrylic copolymer pressure sensitive adhesive, the porous backing being a woven fabric, a knitted fabric, a nonwoven fabric, a melt-blown web, a spun-bonded web, a thermal-bonded web, a spun-laced web, paper, and a thermally-embossed nonwoven fabric,
(b)coating a silicone adhesive gel precursor on the pressure sensitive adhesive, and
(c) curing the silicone adhesive gel precursor;
wherein silicone gel adhesive is formed from a hydrosilation reaction between an alpha-omega vinyl terminated polydimethyl siloxane and a Si-H containing siloxane catalyzed by a platinum catalyst.

8. A method for making a silicone gel adhesive construction comprising:
(a) sealing at least 75% of one side of a porous backing with an acrylic copolymer pressure sensitive adhesive the porous backing being a woven fabric, a knitted fabric, a nonwoven fabric, a melt-blown web, a spun-bonded web, a thermal-bonded web, a spun-laced web, paper, and a thermally-embossed nonwoven fabric, and
(b)laminating a cured silicone gel adhesive to the pressure sensitive adhesive,
wherein the silicone gel adhesive is formed from a hydrosilation reaction between an alpha-omega vinyl terminated polydimethyl siloxane and a Si-H containing siloxane catalyzed by a platinum catalyst.

9. The method of claim 8 wherein the cured silicone gel adhesive is provided on a release liner.

10. The method of claim 7 or 8 wherein the sealing of at least 75% of the one side of the porous backing with pressure sensitive adhesive comprises coating acrylic copolymer on at least 75% of the one side of the porous backing.

11. The method of claim 7 or 8 wherein the sealing of at least 75% of the one side of the porous backing with pressure sensitive adhesive comprises coating acrylic copolymer on a release liner and laminating the acrylic copolymer to at least 75% of the one side of the porous backing.

## Patentansprüche

1. Konstruktion aus Silikongelklebstoff, umfassend:
(a) eine poröse Trägerschicht, die ein gewebter Stoff, ein gestrickter Stoff, ein Vliesstoff, eine schmelzgeblasene Bahn, eine Spinnvliesbahn, eine thermisch gebundene Bahn, eine Spunlaced-Bahn, Papier und ein thermisch geprägter Vliesstoff ist,
(b) eine Acrylcopolymer-Haftklebstoffschicht, die mindestens 75 % einer Seite der porösen Trägerschicht bedeckt und abdichtet, und
(c) einen gehärteten Silikongelklebstoff auf der Haftklebstoffschicht, wobei der Silikongelklebstoff aus einer durch einen Platinkatalysator katalysierten Hydrosilylierungsreaktion zwischen einem alpha-omega mit Vinyl terminierten Polydimethylsiloxan und einem Si-H enthaltenden Siloxan gebildet wird.

2. Konstruktion aus Silikongelklebstoff nach Anspruch 1, wobei die Konstruktion eine Wasserdampfdurchlassrate gemäß ASTM E 96-80 von mindestens 300 g/m²/24 Stunden bei 40 °C aufweist.

3. Konstruktion aus Silikongelklebstoff nach Anspruch 1, wobei das Beschichtungsgewicht der Acrylcopolymer-Haftklebstoffschicht 15 g/m² bis 80 g/m² beträgt.

4. Konstruktion aus Silikongelklebstoff nach Anspruch 1, wobei das Beschichtungsgewicht des Silikongelklebstoffs 20 g/m² bis 150 g/m² beträgt.

5. Konstruktion aus Silikongelklebstoff nach Anspruch 1, wobei der Silikongelklebstoff von 0,02 Millimeter (0,8 mils) bis 0,15 Millimeter (6 mils) dick ist.

6. Konstruktion aus Silikongelklebstoff nach Anspruch 1, wobei der Haftklebstoff zu einem größeren Teil 2-Ethylhexylacrylat oder Butylacrylat und zu einem kleineren Teil Acrylsäure oder Acrylamid umfasst.

7. Verfahren zum Herstellen einer Konstruktion aus Silikongelklebstoff, umfassend:
(a) Abdichten von mindestens 75 % einer Seite einer porösen Trägerschicht mit einem Acrylcopolymer-Haftklebstoff, wobei die poröse Trägerschicht ein gewebter Stoff, ein gestrickter Stoff, ein Vliesstoff, eine schmelzgeblasene Bahn, eine Spinnvliesbahn, eine thermisch gebundene Bahn, eine Spunlaced-Bahn, Papier und ein thermisch geprägter Vliesstoff ist,
(b) Beschichten des Haftklebstoffs mit einem Silikonklebegelvorläufer, und
(c) Härten des Silikonklebegelvorläufers;
wobei der Silikongelklebstoff aus einer durch einen Platinkatalysator katalysierten Hydrosilylierungsreaktion zwischen einem alpha-omega mit Vinyl terminierten Polydimethylsiloxan und einem Si-H enthaltenden Siloxan gebildet wird.

8. Verfahren zum Herstellen einer Konstruktion aus Silikongelklebstoff, umfassend:
(a) Abdichten von mindestens 75 % von einer Seite einer porösen Trägerschicht mit einem Acrylcopolymer-Haftklebstoff, wobei die poröse Trägerschicht ein gewebter Stoff, ein gestrickter Stoff, ein Vliesstoff, eine schmelzgeblasene Bahn, eine Spinnvliesbahn, eine thermisch gebundene Bahn, eine Spunlaced-Bahn, Papier und ein thermisch geprägter Vliesstoff ist, und
(b) Laminieren eines gehärteten Silikongelklebstoffs an den Haftklebstoff, wobei der Silikongelklebstoff aus einer durch einen Platinkatalysator katalysierten Hydrosilylierungsreaktion zwischen einem alpha-omega mit Vinyl terminierten Polydimethylsiloxan und einem Si-H enthaltenden Siloxan gebildet wird.

9. Verfahren nach Anspruch 8, wobei der gehärtete Silikongelklebstoff auf einer Trennschicht bereitgestellt wird.

10. Verfahren nach Anspruch 7 oder 8, wobei das Abdichten von mindestens 75 % der einen Seite der porösen Trägerschicht mit Haftklebstoff das Beschichten von mindestens 75 % der einen Seite der porösen Trägerschicht mit Acrylcopolymer umfasst.

11. Verfahren nach Anspruch 7 oder 8, wobei das Abdichten von mindestens 75 % der einen Seite der porösen Trägerschicht mit Haftklebstoff das Beschichten einer Trennschicht mit Acrylcopolymer und das Laminieren des Acrylcopolymers an mindestens 75 % der einen Seite der porösen Trägerschicht umfasst.

## Revendications

1. Construction adhésive à base de gel de silicone comprenant :
(a) un support poreux étant un tissu tissé, un tissu à mailles, un textile non tissé, une toile soufflée en fusion, une toile filée-liée, une toile liée thermiquement, une toile lacée par filage, un papier et un textile non tissé thermiquement gaufré,
(b) une couche d'adhésif sensible à la pression à base de copolymère acrylique sur, et scellant, au moins 75 % d'un côté du support poreux, et
(c) un adhésif à base de gel de silicone durci sur la couche d'adhésif sensible à la pression, dans laquelle l'adhésif à base de gel de silicone est formé à partir d'une réaction d'hydrosilation entre un polydiméthylsiloxane à terminaison vinylique alpha-oméga et un siloxane contenant Si-H, catalysée par un catalyseur au platine.

2. Construction adhésive à base de gel de silicone selon la revendication 1, où la construction a un taux de transmission de vapeur humide selon ASTM E 96-80 à 40 °C d'au moins 300 g/m²/24 heures.

3. Construction adhésive à base de gel de silicone selon la revendication 1, dans laquelle le poids de revêtement de la couche d'adhésif sensible à la pression à base de copolymère acrylique va de 15 g/m² à 80 g/m².

4. Construction adhésive à base de gel de silicone selon la revendication 1, dans laquelle le poids de revêtement de l'adhésif à base de gel de silicone va de 20 g/m² à 150 g/m².

5. Construction adhésive à base de gel de silicone selon la revendication 1, dans laquelle l'adhésif à base de gel de silicone a une épaisseur de 0,02 millimètre (0,8 mil) à 0,15 millimètre (6 mils).

6. Construction adhésive à base de gel de silicone selon la revendication 1, dans laquelle l'adhésif sensible à la pression comprend une majorité d'acrylate de 2-éthylhexyle ou d'acrylate de butyle et une quantité mineure d'acide acrylique ou d'acrylamide.

7. Procédé de fabrication d'une construction adhésive à base de gel de silicone comprenant :
(a) le fait de sceller au moins 75 % d'un côté d'un support poreux avec un adhésif sensible à la pression à base de copolymère acrylique, le support poreux étant un tissu tissé, un tissu à mailles, un textile non tissé, une toile soufflée en fusion, une toile soufflée en fusion, une toile liée thermiquement, une toile lacée par filage, un papier et un textile non tissé thermiquement gaufré,
(b) le revêtement d'un précurseur de gel adhésif de silicone sur l'adhésif sensible à la pression, et
(c) le durcissement du précurseur de gel adhésif de silicone ;
dans lequel l'adhésif à base de gel de silicone est formé à partir d'une réaction d'hydrosilation entre un polydiméthylsiloxane à terminaison vinylique alpha-oméga et un siloxane contenant Si-H, catalysée par un catalyseur au platine.

8. Procédé de fabrication d'une construction adhésive à base de gel de silicone comprenant :
(a) le fait de sceller au moins 75 % d'un côté du support poreux avec un adhésif sensible à la pression à base de copolymère acrylique, le support poreux étant un tissu tissé, un tissu à mailles, un textile non tissé, une toile soufflée en fusion, une toile filée-liée, une toile liée thermiquement, une toile lacée par filage, un papier et un textile non tissé thermiquement gaufré, et
(b) la stratification d'un adhésif à base de gel de silicone durci sur l'adhésif sensible à la pression, dans lequel l'adhésif à base de gel de silicone est formé à partir d'une réaction d'hydrosilation entre un polydiméthylsiloxane à terminaison vinylique alpha-oméga et un siloxane contenant Si-H, catalysée par un catalyseur au platine.

9. Procédé selon la revendication 8, dans lequel l'adhésif à base de gel de silicone durci est fourni sur une protection détachable.

10. Procédé selon la revendication 7 ou 8, dans lequel le fait de sceller au moins 75 % dudit côté du support poreux avec un adhésif sensible à la pression comprend le revêtement de copolymère acrylique sur au moins 75 % dudit côté du support poreux.

11. Procédé selon la revendication 7 ou 8, dans lequel le fait de sceller au moins 75 % dudit côté du support poreux avec un adhésif sensible à la pression comprend le revêtement de copolymère acrylique sur une protection détachable et la stratification du copolymère acrylique sur au moins 75 % dudit côté du support poreux.
